# FASCICULE DE BREVET EUROPEEN

(11) **EP 0 485 682 B1**
(45) Date de publication et mention de la délivrance du brevet: **16.08.1995**
(21) Numéro de dépôt: 90810876.4
(22) Date de dépôt: 14.11.1990
(51) Int. Cl.: A61L 2/24, A61L 2/22, A61L 9/14

(54) **Installation automatique pour la décontamination économique d'une ou sélectivement de plusieurs enceintes closes**
Automatische Anlage zur wirtschaftlichen Dekontaminierung von einem oder mehreren geschlossenen Räumen
Automatic system for the economical decontamination of one or more closed enclosures

(43) Date de publication de la demande: 20.05.1992
(73) Titulaire: Rast, Francois, Metro Manila (PH)
(72) Inventeur: Petit, Daniel, F-25170 Emagny (FR)
(74) Mandataire: Braun, André, jr.

(56) Documents cités:
- EP-A- 0 291 616
- WO-A-90/12600
- FR-A- 2 302 105
- FR-A- 2 414 337

## Description

L'invention concerne une installation automatique pour la décontamination, la désinfection ou la désodorisation d'une ou individuellement de plusieurs enceintes closes, comme des cabines de toilettes que l'on rencontre par exemple dans des locaux sanitaires publics de grands bâtiments d'administration, de restaurants, d'écoles, d'hôpitaux, d'aérodromes, etc.

Il est connu que les toilettes publiques présentent de très grands risques de contamination de toutes sortes de maladies principalement dues au salissement par les utilisateurs et à la pollution intérieure de ces locaux. Jusqu'il y a peu de temps, on considérait qu'un lavage régulier et une bonne aération suffisent pour entretenir ces lieux d'aisances.

Sans dénigrer la nécessité de lavages réguliers, de récentes recherches démontrent que la ventilation de ce genre de locaux peut à la fois diminuer, mais aussi augmenter la pollution. Une diminution p.ex. est obtenue en ce qui concerne la pollution par le radon ou des formaldehydes. Il a été cependant constaté qu'avec une ventilation la concentration de ces mêmes polluants peut considerablement accroître, si la température du local et l'humidité relative sont élevées.

En ce qui concerne les micro-organismes, comme les bactéries, virus, amibes libres et spores fongiques, il est connu depuis un certain temps qu'ils se développent de manière importante dans des lieux chauffés et encore plus , si ces lieux présentent une humidité élevée et leur transport étant facilité par une ventilation. De l'autre côté, les exigences actuelles de confort demandent à ce que les lieux d'aisances soient bien chauffés. Les locaux de toilettes étant équipés par les lavabos etc., il est évident que ces locaux présentent en général une humidité très élevée, ce qui permet aux microorganismes de se développer dans des conditions idéales. En plus, par la fréquence élevée d'utilisation des toilettes publiques, l'apport complémentaire en bactéries et virus de l'extérieur, augmente le risque de contamination et d'infection en ces lieux à tel point qu'il devient absoluement nécessaire de prévoir des installations de décontamination si on ne veut courir le risque d'épidémies.

Plusieurs inventeurs ont déjà reconnu cette nécessité et ont proposé diverses solutions pour éliminer ou au moins diminuer le risque de contamination. Le brevet français 1.594.677 révèle un dispositif provoquant automatiquement un jet d'aérosol à la fermeture d'une porte de toilette. On propose d'employer à cet effet une bombe du type aérosol montée dans un support au cadre au-dessus de la porte et un organe fixe sur l'ouvrant qui appuie sur le bouton-poussoir de la bombe chaque fois que l'on ferme la porte. Bien que ce brevet ne prévoit que l'emploi d'un produit désodorant, il est évident que l'invention pourrait également servir à la projection d'un produit décontaminant ou désinfectant.

Cette solution présente toutefois de gros désavantages. Il se peut qu'une personne ne ferme pas complètement la porte, ce qui peut résulter en une vidange complète de la bombe aérosol. D'autre part, il est inévitable que le dispositif provoque aussi un jet d'aérosol lorsque une personne ferme la porte après s'être introduite dans la cabine. Cette aspersion n'est certainement pas désirée et représente en plus une consommation inutile en produit assainissant.

La demande de brevet français no 78 00865 relève un procédé, selon lequel on injecte dans l'atmosphère du milieu à décontaminer une solution liquide bactéricide sous forme de gouttelettes ou particules et pulverisée à partir d'une pompe hydraulique, la solution sous pression traversant une buse d'éjection apte à assurer la formation des particules et la projection de ces dernières dans le milieu ambiant. Le procédé et les dispositifs cités dans cette demande peuvent parfaitement se prêter pour une décontamination occasionelle ou même régulière de locaux infectés, mais ils ne conviennent pas à la décontamination rapide, individuelle et économique de chaque cabine de toilette utilisée dans un grand local sanitaire public.

Les principaux inconvenients de ce procédé et des dispositifs proposés consistent d'une part dans le fait que la buse d'injection est directement desservie par la pompe hydraulique, ce qui necessite que pour chaque éjection la pompe soit ou se mette en marche, et d'autre part que le débit de la buse d'injection n'est pas ou du moins que difficilement contrôlable d'une manière précise.

La demande de brevet WO-A-9012600 révèle une méthode et l'installation pour la désinfection spécialement de locaux d'usines alimentaires. Cette invention propose un procédé de désinfection se composant de plusieurs actions: a) d'une humidification du local par atomisation de l'eau jusqu'à ce que toutes les surfaces rigides soient couvertes d'une couche d'eau; ensuite b) d'une atomisation d'un désinfectant dans le local; ensuite c) d'une pause pour laisser agir le produit désinfectant dans le local; et finalement d) d'une évacuation par rinçage du désinfectant. Les 4 actions successives de cette méthode nécessitent relativement beaucoup de temps. En plus, il en résulte, que toutes les surfaces sont mouillées ce qui p. ex. pour une désinfection rapide nécessaire aux toilettes de locaux sanitaires publics fréquentées à courtes intervalles, ne peut donner satisfaction. L'installation proposée par la WO-A-9012600 est sans doute principalement destinée à des locaux dans des bâtiments d'usine et ne se prête non-plus pas p.ex. à un emploi dans des locaux mobiles, comme des avions, trains ou cars, vu qu'elle doit obligatoirement être branchée sur une amenée d'eau, être équipé d'une conduite d'air comprimé et que le sol du local à assainir doit être pourvu d'une évacuation d'eau.

La tâche de la présente invention consiste dans le développement d'une installation automatisée et spécialisée pour la décontamination, la désinfection ou la désodorisation efficace et économique d'une ou individuellement de plusieurs cabines de toilettes dans de grands locaux sanitaires publics et ceci tout en réduisant au stricte minmum la consommation en produit assainissant et en énergie.

Cette tâche est résolue par une installation automatique pour la décontamination, désinfection ou désodorisation d'une ou de plusieurs enceintes closes dans un local ou d'un ou de plusieurs locaux, l'installation se composant d'un appareil central comprenant un tableau de commande et un réservoir pourvu d'un dispositif de compression, et de moyens appropriés pour la pulvérisation d'un produit assainissant, avec les caractéristiques suivantes:
- le réservoir contenant le produit assainissant est relié aux moyens de pulvérisation par une unique tubulure se ramifiant selon le nombre d'enceintes, cellules ou locaux qui sont à desservir;
- le dispositif de compression exerce une surpression relativement faible et constante sur le produit assainissant contenu dans le réservoir et dans l'unique tubulure;
- chaque moyen de pulvérisation étant composé d'une électro-vanne et d'un pulvérisateur et commandé par un interrupteur ou détecteur installé dans chaque enceinte, cellule ou local.

Un des avantages essentiels de l'invention consiste dans le fait que par un dispositif de compression, le produit assainissant dans le réservoir et la tubulure est constamment sous une surpression relativement faible, ce qui rend possible que la pulvérisation du produit assainissant se fasse intantanément par simple ouverture des électro-vannes, chacune pouvant être commandée directement ou indirectement par un interrupteur ou détecteur électrique signalant l'utilisation d'une enceinte, d'une cellule ou d'un local. Chaque enceinte, cellule ou local disposant de son propre moyen de pulvérisation et de son propre interrupteur ou détecteur, l'action d'assainissement se limite à l'enceinte utilisée.

L'installation fontionnant à relativement basse pression (de l'ordre de 2 à 3 bars pour des locaux sanitaires avec env. 4-8 cabines), la réserve de surpression obtenue par le dispositif de compression est suffisante pour assurer un grand nombre de pulvérisations avant qu'un rétablissement de la surpression initiale devienne nécessaire, ce qui diminue considérablement la consommation en énergie.

L'installation étant groupée en un appareil central du type monobloc et en une tubulure à une ou plusieurs ramifications avec les moyens de pulvérisation, le lieu de montage de l'appareil central est sans importance; l'appareil central peut être monté dans le local à desservir ou dans un local indépendant, p.ex. un local de service, ce qui facilite son entretien et limite par la même occasion les perturbations des utilisateurs par les bruits éventuels provoqués lors du rétablissement de la surpression par le dispositif de compression.

Dans une version d'exécution simple, un détecteur électrique alimenté depuis le tableau de commande de l'appareil central et p.ex. équipé d'un temporisateur réglant la durée d'ouverture de l'électro-vanne, commande directement l'électrovanne afférente au détecteur électrique de l'enceinte utilisée. Dans une exécution plus perfectionnée, la commande des électrovannes se fait indirectement, c'est-à-dire que les détecteurs électriques transmettent leurs détections au tableau de commande de l'appareil central, d'où l'ouverture et la fermeture de l'électro-vanne correspondante sont ordonnées.

Le nombre de ramifications, chacune équipée d'un moyen de pulvérisation, que présente l'installation, n'est indirectement limité que par la pression qui peut être obtenue par le dispositif de compression et par la pression nécessaire au bon fonctionnement de l'installation.

Le dispositif de compression exerçant une surpression sur le produit assainissant peut être une vessie prégonflée se trouvant à l'intérieur du réservoir contenant le produit assainissant. Un autre dispositif de compression consiste en un piston monté sur ou dans le réservoir qui excerce une surpression directe sur le produit assainissant, le piston étant entraîné mécaniquement ou p.ex. par air comprimé.

Etant donné que l'installation fonctionne à relativement basse pression et que la quantité volumétrique de produit assainissant nécessaire pour une pulvérisation est minime, la tubulure et toutes les pièces raccordées (moyens de pulvérisation, pièces de raccordement, etc.) de l'installation peuvent présenter des dimensions minimales, ce qui permet un montage peu voyant et discrèt dans les cabines.

D'autre caractéristiques de l'invention font partie de revendications particulières et seront précisées avec d'autres avantages de l'invention dans la description des exemples de réalisation.

Les dessins représentent en exemples deux exécutions de l'installation selon l'invention. Ils montrent
- Fig. 1: le schéma d'une installation simple;
- Fig. 2: le schéma d'une installation desservant un local avec plusieurs enceintes à assainir, l'appareil central se trouvant dans un autre local.

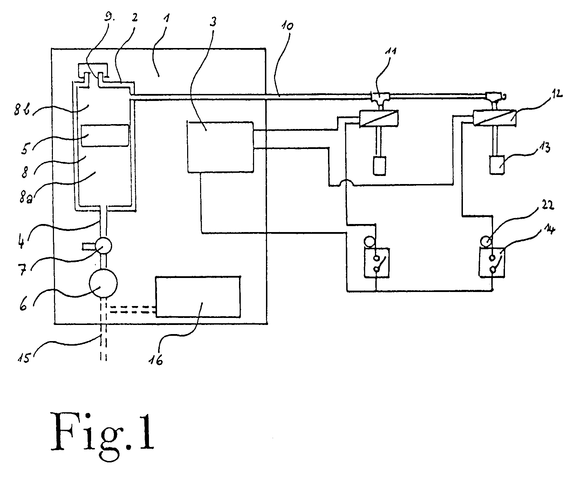

La figure 1 représente les parties essentielles d'une exécution suivant l'invention. L'installation se compose principalement de l'appareil central 1 et de la tubulure 10 qui présente une ou plusieurs ramifications 11, chacune étant équipée d'une électro-vanne 12 et d'un pulvérisateur 13. L'électro-vanne 12 est alimentée en courant électrique par le tableau de commande 3; le détecteur électrique 14, ici représenté comme un interrupteur pouvant être monté p.ex. dans la serrure de la porte de l'enceinte 23, 24, commande l'ouverture et la fermeture de l'électro-vanne 12, et détermine la durée d'ouverture s'il est pourvu d'un temporisateur 22.

L'appareil central se compose d'un tableau de commande 3 et d'un réservoir 2 pourvu d'un dispositif de compression 4, lequel dans une version simple prévoit à ce que l'espace 8a de la cavité 8 du réservoir 2 est formée à recevoir le piston 5 entraîné par de l'air comprimé et qui exerce une surpression sur le produit assainissant 7 contenu dans l'espace 8b de la cavité 8 du réservoir 2 et dans la tubulure 10. La réalimentation en produit assainissant 7 se fait par une tubulure de remplissage 9 qui est fermée lorsque l'installation est en marche.

La pression de l'air comprimé agissant sur le piston 5 est réglable par un régulateur de pression 6. Pour la réalimentation du réservoir 2 en produit assainissant 7, la pression dans l'espace 8a de la cavité 8 du réservoir 2 est évacuée par la vanne de dérivation 7, le régulateur de pression 6 étant fermé. Pour augmenter l'automatisme de cette exécution d'installation, le régulateur de pression 6 et la vanne de dérivation 7 sont avantageusement électrifiés et raccordés au tableau de commande 3 qui, étant équipé en conséquence, est à même d'ordonner automatiquement le fonctionnement et l'ordre logique des différents éléments (l'équipement du tableau de commande et son raccordement à celui-ci ne sont pas dessinés).

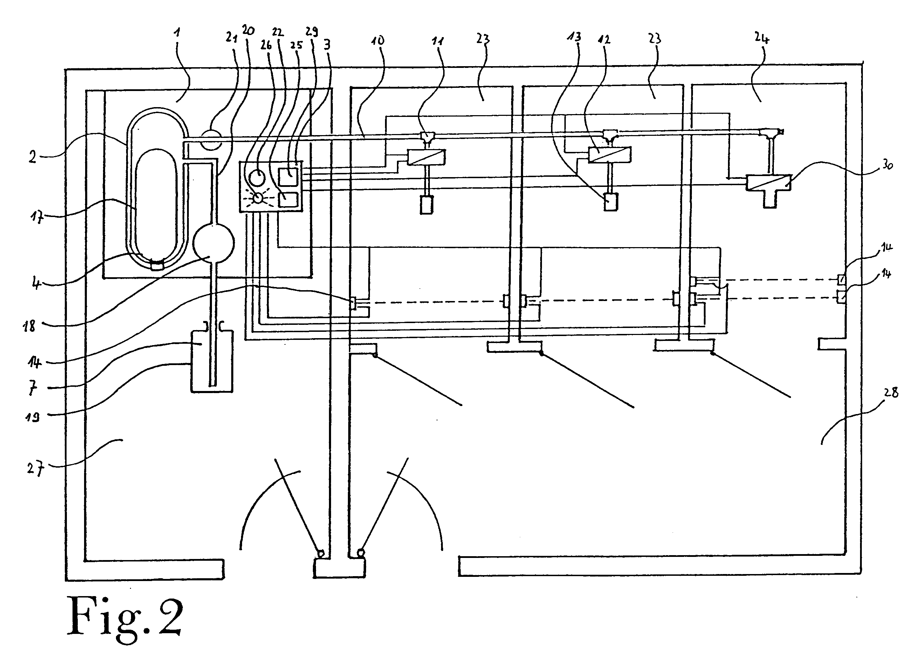

La figure 2 montre le schéma d'une installation très perfectionnée desservant plusieurs enceintes dans un local et l'appareil central se trouvant dans un local séparé. Cette installation comporte également toutes les parties essentielles qui sont complétées par divers accessoires augmentant l'automatisme et le confort de l'installation. Pour cette exécution il est proposé un dispositif de compression 4 altérnatif qui consiste en une vessie prégonflée 17 se trouvant dans l'espace 8a du réservoir 2. Une pompe hydraulique 18 aspire le produit assainissant 7 d'un récipient indépendant 19 et le transporte par la canalisation 20 dans le réservoir 2, l'action de pompage ne s'arrêtant qu'à l'instant où dans le réservoir 2 et dans la tubulure 10 la surpression désirée est atteinte.

Il est évident, que par un régulateur de pression 21 placé à la sortie du réservoir 2 au début de la tubulure 10 il est possible de maintenir une surpression supérieure dans le réservoir 2 par rapport à celle dans la tubulure 10.

Selon cette exécution, le détecteur électrique 14 est alimenté en courant électrique par le tableau de commande 3 auquel il signale ses détections. C'est le tableau de commande 3 qui ordonne l'ouverture et la fermeture de l'électro-vanne 12 afférente au détecteur 14 émettant les signaux. Le tableau de commande 3, étant équipé d'un temporisateur 22, détermine la durée de l'ouverture de l'électro-vanne 12.

Dans cette figure, les détecteurs électriques 14 installés dans les enceintes 23 représentent des cellules photoélectriques simples. En doublant le détecteur électrique 14 par un deuzième identique, comme montré dans l'enceinte 24, ou en employant alternativement une cellule photoélectrique à double détection, le module programmable 25 incorporé dans le tableau de commande 3 distingue l'ordre de leurs signaux, ce qui lui permet de supprimer l'ouverture de l'électro-vanne 12 à l'entrée d'un utilisateur de l'enceinte 24.

Le même module programmable 25 peut arrêter le fonctionnement de chaque élément électrique ou de toute l'installation en cas de panne et avertir le responsable de l'entretien p.ex. moyennant des signaux lumineux intermittents 26.

Pour faciliter l'entretien de l'appareil central et surtout dans le but d'éliminer du lieu d'aisance les bruits que peut produire le dispositif de compression 4 lorsque celui-ci se met en marche, l'appareil central n'est pas placé dans le local 28 où sont placées les enceintes 23,24,mais dans un local spécial de service 27.

Pour diminuer le risque d'électrocution, le tableau de commande 3 est pourvu d'un transformateur 29 alimentant le circuit du module programmable 25, les détecteurs électriques 14 et les électro-vannes 12 à un voltage inférieur à celui nécessaire pour le dispositif de compression 4.

Pour des raison de rationalisation du montage, il est avantageux de coupler directement le pulvérisateur 13 à l'électro-vanne 12 pour former un boîtier d'une pièce 30. Pour les même raisons, il est profitable que les raccordements hydrauliques entre le réservoir 2 et la tubulure 10, entre les ramifications 11 et les électro-vannes 12 et les pulvérisateurs 13 ainsi que le branchement entre les différents éléments électriques et les cables correspondants, sont des connexions à ficher (pas déssiné).

## Revendications

1. Installation automatique pour la décontamination, désinfection ou désodorisation d'une ou de plusieurs enceintes closes dans un local ou d'un ou plusieurs locaux, l'installation se composant d'un appareil central comprenant un tableau de commande et un réservoir pourvu d'un dispositif de compression, et de moyens appropriés pour la pulvérisation d'un produit assainissant,
**caractérisée** en ce que
- le réservoir contenant le produit assainissant est relié aux moyens de pulvérisation par une unique tubulure se ramifiant selon le nombre d'enceintes, cellules ou locaux à desservir;
- le dispositif de compression exerce une surpression relativement faible et constante sur le produit assainissant contenu dans le réservoir et dans l'unique tubulure;
- chaque moyen de pulvérisation étant composé d'une électro-vanne et d'un pulvérisateur et commandé par un interrupteur ou détecteur installé dans chaque enceinte, cellule ou local.

2. Installation selon la revendication 1, caractérisée en ce que la pulvérisation d'un produit assainissant se fait instantanément par simple ouverture d'une l'électro-vanne commandée par un interrupteur ou un détecteur électrique singnalant l'utilisation de l'enceinte;

3. Installation selon les revendications 1 et 2, caractérisée en ce que la réserve de surpression dans le réservoir et la tubulure est suffisante pour un grand nombre de pulvérisations;

4. Installation selon les revendications 1 à 3, caractérisée en ce que le dispositif de compression consiste en une vessie prégonflée ou un piston se trouvant à l'intérieur du réservoir exerçant directement une pression sur le produit assainissant;

5. Installation selon les revendications 1 à 4, caractérisée en ce que l'appareil central comporte un compresseur ou une pompe hydraulique rétablissant la surpression sur le moyen assainissant;

6. Installation selon les revendications 1 à 5, caractérisée en ce que le dispositif de compression est branché, avec des moyens contrôlant la surpression et permettant la réalimentation en moyen assainissant, sur une source indépendante d'air comprimé;

7. Installation selon les revendications 1 à 6, caractérisée en ce que le détecteur électrique peut consister en une cellule photoélectrique ou un autre détecteur de ce genre pouvant signaler l'utilisation de l'enceinte, de la cellule ou du local où le détecteur est installé;

8. Installation selon les revendications 1 à 7, caractérisée en ce que l'électro-vanne et le pulvérisateur sont directement couplés pour former un boîtier unique;

9. Installation selon les revendications 1 à 8, caractérisée en ce que le tableau de commande est équipé d'un temporisateur réglable commandant la durée d'ouverture des électro-vannes et par ceci la quantité pulvérisée en produit assainissant;

10. Installation selon les revendications 1 à 9, caractérisée en ce que le produit assainissant consiste en des solutions protégeant des bactéries, amibes libres, virus et spores fongiques;

11. Installation selon les revendications 1 à 10, caractérisée en ce que des parfums sont ajoutés au produit assainissant;

12. Installation selon les revendications 1 à 11, caractérisée en ce que les raccordements entre le réservoir et la tubulure, entre les ramifications et les moyens de pulvérisation ainsi que les raccordements de conduites électriques raccordant les différents éléments, sont des connexions à ficher.

## Claims

1. An automatic plant for the decontamination, disinfection or deodorisation of one or several closed rooms in premises or of one or several premises, the plant consisting of a central apparatus that comprises a control console and a container provided with a compression device and appropriate means for the atomisation of a cleansing agent, characterised in that
- the container containing the cleansing agent is associated with the atomising means by means of a single conduit which is branched depending on the number of rooms, cells or premises to be served;
- each atomising means being composed of an electrovalve and an atomiser and controlled by a switch or a detector installed in each room, cell or premises.

2. A plant according to Claim 1, characterised in that the atomisation of the cleansing agent occurs immediately, simply by opening the electrovalve by means of a switch or a detector signalling the use of the room.

3. A plant according to Claims 1 and 2, characterised in that the excess pressure reserve in the container and the conduit is sufficient for a large number of atomisations.

4. A plant according to Claims 1 to 3, characterised in that the compression device consists of an inflated bellows or a piston disposed in the container which exerts direct pressure on the cleansing agent.

5. A plant according to Claims 1 to 4, characterised in that the central apparatus comprises a compressor restoring the excess pressure on the cleansing agent or a hydraulic pump.

6. A plant according to Claims 1 to 5, characterised in that the compression device with the means that control the excess pressure and permit the topping up of cleansing agent are connected to an independent source of compressed air.

7. A plant according to Claims 1 to 6, characterised in that the electrical detector may consist of a photocell or another detector of this type which can signal the use of a room, a cell or a premises where the detector is installed.

8. A plant according to Claims 1 to 7, characterised in that the electrovalve and the atomiser are directly coupled and form a single housing.

9. A plant according to Claims 1 to 8, characterised in that the control console is equipped with an adjustable time switch that controls the opening time of the electrovalves and hence also the amount of cleansing agent atomised.

10. A plant according to Claims 1 to 9, characterised in that the cleansing agent consists of solutions that protect against bacteria, free amoebas, viruses and fungus spores.

11. A plant according to Claims 1 to 10, characterised in that perfumes are added to the cleansing agent.

12. A plant according to Claims 1 to 11, characterised in that the connections between container and conduit, between the branches and the atomising means as well as the connections of the electrical leads which connect the various elements are connector assemblies.

## Patentansprüche

1. Automatische Anlage für die Dekontaminierung, Desinfektion oder Desodorierung eines oder mehrerer abgeschlossener Räume in einem Lokal oder von einem oder mehreren Lokalen, wobei die Anlage aus einem zentralen Gerät, das ein Steuerpult und einen mit einer Kompressionsvorrichtung versehenen Behälter umfasst und geeigneten Mitteln für die Zerstäubung eines Reinigungsproduktes besteht, dadurch **gekennzeichnet**, dass
- der das Reinigungsprodukt enthaltende Behälter mit den Zerstäubungsmitteln durch eine einzige Rohrleitung verbunden ist, welche sich je nach Anzahl der zu bebetreuenden Räume, Zellen oder Lokale, verzweigt;
- jedes Zerstäubungsmittel sich aus einem Elektroventil und einem Zerstäuber zusammensetzt und durch einen Schalter oder einen Detektor, der in jedem Raum, Zelle oder Lokal installiert ist, gesteuert wird.

2. Anlage nach Anspruch 1, dadurch gekennzeichnet, dass die Zerstäubung des Reinigungsproduktes unverzüglich bloss durch Öffnung des Elektroventils durch einen Schalter oder einen die Benützung des Raumes signalisierenden Detektor erfolgt;

3. Anlage nach den Ansprüchen 1 und 2, dadurch gekennzeichnet, dass die Überdruckreserve im Behälter und der Rohrleitung für eine grosse Anzahl von Zerstäubungen ausreicht;

4. Anlage nach den Ansprüchen 1 bis 3, dadurch gekennzeichnet, dass die Kompressionsvorrichtung in einer aufgepumpten Blase oder einem im Behälter befindlichen Kolben besteht, der einen direkten Druck auf das Reinigungsprodukt ausübt;

5. Anlage nach den Ansprüchen 1 bis 4, dadurch gekennzeichnet, dass das zentrale Gerät einen den Überdruck auf das Reinigungsprodukt wiederherstellenden Kompressor oder eine hydraulische Pumpe umfasst;

6. Anlage nach den Ansprüchen 1 bis 5, dadurch gekennzeichnet, dass die Kompressionsvorrichtung mit den Überdruck kontrollierenden und das Nachfüllen vom Reinigungprodukt ermöglichenden Mitteln, an eine unabhängige Druckluftquelle angeschlossen ist;

7. Anlage nach den Ansprüchen 1 bis 6, dadurch gekennzeichnet, dass der elektrische Detektor in einer Photozelle oder einem andern Detektor dieser Art bestehen kann, der die Benützung eines Raumes, einer Zelle oder eines Lokales, wo der Detektor installiert ist, melden kann;

8. Anlage nach den Ansprüchen 1 bis 7, dadurch gekennzeichnet, dass das Elektroventil und der Zerstäuber direkt gekoppelt sind und ein einziges Gehäuse bilden;

9. Anlage nach den Ansprüchen 1 bis 8, dadurch gekennzeichnet, dass das Steuerpult mit einem regulierbaren, die Öffnungsdauer der Elektroventile und damit auch der Menge des zerstäubten Reinigungsproduktes steuernden Zeit-Schalter ausgestattet ist;

10. Anlage nach den Ansprüchen 1 bis 9, dadurch gekennzeichnet, dass das Reinigungsprodukt aus Lösungen besteht, die vor Bakterien, freien Amöben, Viren und Pilzsporen schützen;

11. Anlage nach den Ansprüchen 1 bis 10, dadurch gekennzeichnet, dass dem Reinigungsprodukt Parfums beigefügt sind;

12. Anlage nach den Ansprüchen 1 bis 11, dadurch gekennzeichnet, dass die Verbindungen zwischen Behälter und Rohrleitung, zwischen den Verzweigungen und den Mitteln für die Zerstäubung sowie die Verbindungen der elektrischen Leitungen, welche die verschiedenen Elemente verbinden, Steckverbindungen sind.
